# EUROPÄISCHE PATENTSCHRIFT

(11) **EP 0 463 477 B1**
(45) Veröffentlichungstag und Bekanntmachung des Hinweises auf die Patenterteilung: **16.03.1994**
(21) Anmeldenummer: 91109638.6
(22) Anmeldetag: 12.06.1991
(51) Int. Cl.: C09B 25/00

(54) **Verfahren zur Herstellung von Chinophthalonen**
Method of preparation of quinophthalones
Procédé de préparation de quinophthalones

(30) Priorität: 27.06.1990 DE 4020423
(43) Veröffentlichungstag der Anmeldung: 02.01.1992
(73) Patentinhaber: BASF Aktiengesellschaft, 67063 Ludwigshafen (DE)
(72) Erfinder: Ort, Burkhard, Dr., W-6706 Wachenheim (DE); Kuth, Guido, Dr., W-6700 Ludwigshafen (DE)

(56) Entgegenhaltungen:
- DE-A- 1 770 960
- DE-A- 2 230 601
- GB-A- 1 091 734

## Beschreibung

Die vorliegende Erfindung betrifft ein neues Verfahren zur Herstellung von Chinophthalonen durch Kondensation von 8-Aminochinaldin mit gegebenenfalls durch Chlor oder Brom ein- oder mehrfach substituiertem Phthalsäureanhydrid in Gegenwart eines Verdünnungsmittels.

In Chimia 24, 328, 1970 sowie in der GB-A-1 091 734 wird vorgeschlagen, die Kondensation von 2-Methylchinolinderivaten (Chinaldinderivaten) mit aromatischen ortho-Dicarbonsäuren oder deren Anhydriden in Gegenwart von inerten, hochsiedenden Verdünnungsmitteln, wie ortho-Dichlorbenzol, Tri-chlorbenzol, Nitrobenzol, Naphthalin, Diphenyl oder Diphenylether, vorzunehmen.

Weiterhin wird in der DE-A-1 770 960 sowie in der DE-A-2 230 601 die zusätzliche Verwendung von Lewis- oder Brönstedt-Säuren zur Beschleunigung der Kondensation empfohlen.

Diese Verfahren weisen aber Nachteile auf. So ist in der Regel eine Reaktionstemperatur erforderlich, die größer als 200°C ist. Dies kann vor allem bei der Herstellung hochreiner Gelbpigmente, z.B. bei der Herstellung des Kondensationsprodukts aus 1 Mol 8-Aminochinaldin mit 2 Mol Tetrachlorphthalsäureanhydrid, infolge teilweiser Zersetzung der Aminokomponente zu unerwünschten Abtrübungen führen, was gegebenenfalls aufwendige Nachreinigungsschritte erfordert. Um Pigmente mit hoher Brillanz zu erhalten, ist es erforderlich mit einem Überschuß an Tetrachlorphthalsäureanhydrid (ca. 30 bis 50 Mol %) zu arbeiten. Dieses überschüssige Tetrachlorphthalsäureanhydrid geht in der Regel bei der Regenerierung des verwendeten Verdünnungsmittels verloren. Außerdem sind einige der vorgeschlagenen Verdünnungsmittel, z.B. Nitrobenzol oder chlorierte Benzole, ökologisch nicht unbedenklich.

Aufgabe der vorliegenden Erfindung war es daher, ein neues Verfahren zur Herstellung von Chinophthalonen bereitzustellen, das die obengenannten Schwierigkeiten nicht mehr aufweist und technisch einfach durchzuführen ist. Außerdem sollten die Verfahrensprodukte in guter Ausbeute und hoher Reinheit anfallen.

Es wurde nun gefunden, daß die Herstellung von Chinophthalonen der Formel I 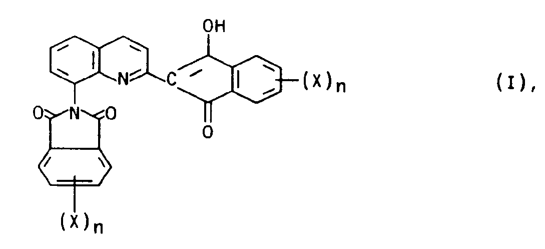
in der X Wasserstoff, Chlor oder Brom und n eine Zahl von 1 bis 4 bedeuten, durch Kondensation von 8-Aminochinaldin der Formel II 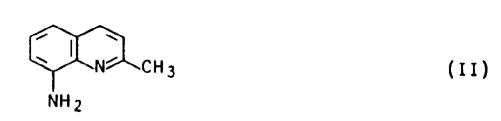
mit einem Phthalsäureanhydrid der Formel III 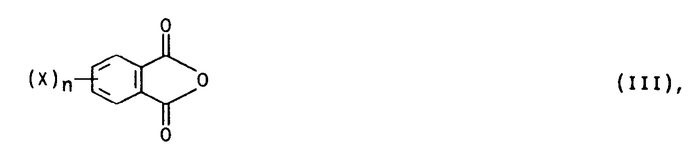
in der X und n jeweils die obengenannte Bedeutung besitzen, in Gegenwart eines Verdünnungsmittels vorteilhaft gelingt, wenn man geschmolzene Benzoesäure als Verdünnungsmittel verwendet.

Für das erfindungsgemäße Verfahren geeignete Phthalsäureanhydride der Formel III sind z.B. Phthalsäureanhydrid, Mono-, Di-, Tri- oder Tetrachlorphthalsäureanhydrid oder Mono-, Di-, Tri- oder Tetrabromphthalsäureanhydrid.

Die Verwendung von Tetrachlorphthalsäureanhydrid (X = Chlor, n = 4) ist bevorzugt.

Das erfindungsgemäße Verfahren, das sowohl in kontinuierlicher als auch in diskontinuierlicher Arbeitsweise vorgenommen werden kann, wird zweckmäßig bei Normaldruck oder bei leicht erhöhtem Druck (bis zu ca. 6 bar) durchgeführt. Als Reaktionsapparaturen im industriellen Maßstab können z.B. an sich gebräuchliche Rührkessel oder auch selbstreinigende Apparate mit Zwangsdurchmischung verwendet werden. Man legt in der Regel feste Benzoesäure vor und schmilzt diese auf. (Der Schmelzpunkt der Benzoesäure beträgt 122,3°C.)

Danach gibt man unter Rühren 8-Aminochinaldin und Phthalsäureanhydrid III zu und erhitzt auf eine Temperatur von 123 bis 200°C, vorzugsweise 130 bis 170°C.

Von besonderem Vorteil ist es, Benzoesäure und Phthalsäureanhydrid III vorzulegen und anschließend 8-Aminochinaldin bei der erfindungsgemäßen Temperatur in einem Zeitraum von 10 bis 180 Minuten zuzudosieren.

Eine weitere bevorzugte Variante des erfindungsgemäßen Verfahrens besteht darin, vor Beginn der Umsetzung Benzoesäure, 8-Aminochinaldin und Phthalsäureanhydrid III vor dem Erwärmen intensiv zu vermischen. Nach dieser Methode werden besonders reine Chinophthalone erhalten.

Bei der Erwärmung des Reaktionsgemisches ist es weiterhin von Vorteil, diesen Schritt in zwei Stufen vorzunehmen. So kann das Reaktionsgemisch zunächst auf eine Temperatur von 130 bis 150°C erhitzt und ca. 1 bis 3 Stunden bei dieser Temperatur gehalten werden, bevor die weitere Erwärmung auf eine Temperatur von 150 bis 180°C erfolgt.

8-Aminochinaldin und Phthalsäureanhydrid III werden im allgemeinen im Molverhältnis 1 : 2 bis 1 : 3 angewandt.

Je Mol 8-Aminochinaldin verwendet man im allgemeinen 0,5 bis 20 Mol, vorzugsweise 2 bis 15 Mol, an Benzoesäure.

Verglichen mit den Verfahren des Standes der Technik ist im erfindungsgemäßen Verfahren die Reaktionstemperatur deutlich erniedrigt und die Reaktionszeit verkürzt. Dies deutet darauf hin, daß die Benzoesäure neben ihrer Eigenschaft als Verdünnungsmittel gleichzeitig die Kondensationsreaktion beschleunigt.

Die Zugabe von speziellen Katalysatoren zur Reaktionsbeschleunigung bringt daher in der Regel keine weiteren Vorteile.

Nach Beendigung der Kondensation, die im allgemeinen 3 bis 8 Stunden in Anspruch nimmt, wird das Reaktionsgemisch auf eine Temperatur von 130 bis 150°C abgekühlt, gegebenenfalls mit Wasser verdünnt und anschließend mit Alkalilauge, z.B. 5 bis 20 gew.-%ige Natronlauge oder Kalilauge, versetzt. Dabei wird die Benzoesäure in ein wasserlösliches Alkalibenzoat übergeführt und die Chinophthalone der Formel I können durch Filtration abgetrennt und mit Wasser gewaschen werden.

Aus der Mutterlauge kann die Benzoesäure durch Ansäuern ausgefällt, isoliert und nahezu ohne Verlust erneut für die Kondensation verwendet werden. Andererseits ist Benzoesäure jedoch auch gut abbaubar. Zusammen mit der Benzoesäure kann auch überschüssiges Phthalsäureanhydrid III nahezu quantitativ zurückgenommen werden.

Eine andere Möglichkeit der Aufarbeitung besteht z.B. darin, das Reaktionsgemisch in einem inerten organischen Lösungsmittel, wie Methanol, Ethanol, Propanol, Isopropanol, Ethylenglykolmonobutylether, Toluol, Xylol oder deren Gemische, aufzunehmen. Dabei wird die Benzoesäure gelöst und die Chinophthalone der Formel I können durch Filtration abgetrennt und mit dem Lösungsmittel gewaschen werden. Durch Abdampfen des Lösungsmittels kann die Benzoesäure zurückgewonnen werden.

Es ist auch möglich, die zurückgewonnene wasserfeuchte Benzoesäure, die gegebenenfalls noch die dem Phthalsäureanhydrid III zugrundeliegende Phthalsäure enthält, direkt als wasserfeuchten Preßkuchen in das erfindungsgemäße Verfahren einzubringen. In diesem Fall wird der eigentlichen Kondensation ein Entwässerungsschritt vorangestellt, um das Reaktionsgemisch zu entwässern und die Phthalsäure in Phthalsäureanhydrid überzuführen.

Es ist weiterhin auch möglich, anstelle von Phthalsäureanhydrid III die entsprechende Phthalsäure als Ausgangsprodukt zu verwenden. Auch in diesem Falle wird ein Entwässerungsschritt vorangestellt, in dem die Anhydridbildung erfolgt.

Das neue Verfahren liefert die Chinophthalone der Formel I in guter Ausbeute und hoher Reinheit.

Die mittels des erfindungsgemäßen Verfahrens erhaltenen Chinophthalone der Formel I sind wertvolle Pigmente.

Die folgenden Beispiele sollen die Erfindung näher erläutern.

### Beispiel 1

In einem Rührkessel wurden 270 g Benzoesäure vorgelegt und aufgeschmolzen. In die Schmelze wurden zwischen 125°C und 130°C nacheinander 82 g Tetrachlorphthalsäureanhydrid und 19 g 8-Aminochinaldin eingetragen. Man erhitzte auf 140°C, rührte 2 Stunden nach, erhitzte dann weiter auf 160°C und rührte weitere 2 Stunden nach. Anschließend kühlte man auf 130°C und versetzte das Reaktionsgemisch mit einer Mischung aus 400 ml Wasser und 160 g Natronlauge (50 gew.-%ig). Man filtrierte die Pigmentsuspension über eine Nutsche, wusch mit Wasser neutral und trocknete. Man erhielt 82 g (98 %) Chinophthalon der Formel

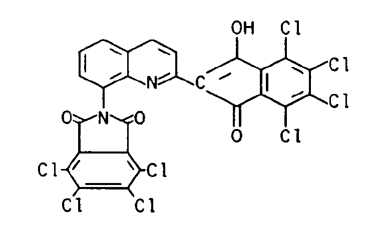

### Beispiel 2

Man verfuhr analog Beispiel 1, verwendete jedoch 122 g Tetrabromphthalsäureanhydrid. Ausbeute: 120 g (95 %) Chinophthalon der Formel

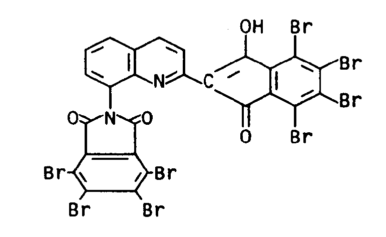

### Beispiel 3

Die aus der Mutterlauge von Beispiel 1 durch Ansäuern und Ausfällen quantitativ zurückgewonnene wasserfeuchte Benzoesäure enthielt 13 g Tetrachlorphthalsäure. Dieser Preßkuchen wurde im Reaktionskessel aufgeschmolzen und solange Wasser abdestilliert, bis die Innentemperatur 115°C erreichte. In die Schmelze wurden dann nacheinander 69 g Tetrachlorphthalsäureanhydrid und 19 g 8-Aminochinaldin eingetragen. Das weitere Vorgehen erfolgte analog Beispiel 1. Ausbeute: 80 g (96 %) Chinophthalon der Formel

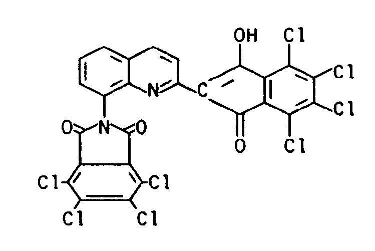

### Beispiel 4

270 g Benzoesäure und 80 g Tetrachlorphthalsäure wurden in einem Rührkessel vorgelegt und aufgeschmolzen. Man erhitzte unter Abdestillieren von Wasser auf 130°C und rührte nach, bis kein Wasser mehr überging (ca. 2 Stunden). Man trug dann 19 g 8-Aminochinaldin ein und erhitzte auf 140°C. Das weitere Vorgehen erfolgte analog Beispiel 1. Ausbeute: 82 g (98 %) Chinophthalon der Formel

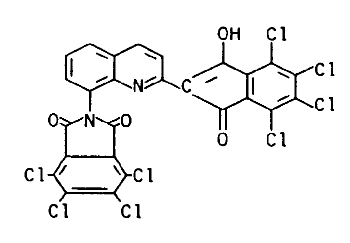

### Beispiel 5

270 g Benzoesäure, 82 g Tetrachlorphthalsäureanhydrid und 19 g 8-Aminochinaldin wurden intensiv vermischt und das Gemisch in einem Rührkessel aufgeschmolzen. Man ließ 2 Stunden bei 135°C reagieren, erhitzte auf 160°C und rührte 2 Stunden nach. Die Aufarbeitung erfolgte analog Beispiel 1.
Ausbeute: 82 g (98 %) Chinophthalon der Formel

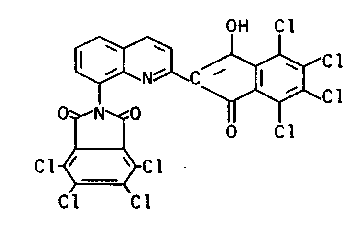

### Beispiel 6

In einem Schaufeltrockner wurden 40 g Benzoesäure, 19 g 8-Aminochinaldin und 46 g Tetrachlorphthalsäureanhydrid vorgelegt, intensiv vermischt und durch Aufheizen aufgeschmolzen. Man erhitzte langsam auf 180°C und hielt diese Temperatur 10 Stunden lang. Anschließend wurde auf 120°C abgekühlt und mit ca. 600 g Natronlauge (3 gew.-%ig) versetzt. Die Suspension wurde ausgetragen, durch Zugabe von verdünnter Natronlauge ein pH-Wert von 10,5 eingestellt, das Pigment abfiltriert, mit Wasser neutral gewaschen und getrocknet.

Man erhielt 81 g (97 %) Chinophthalon der Formel

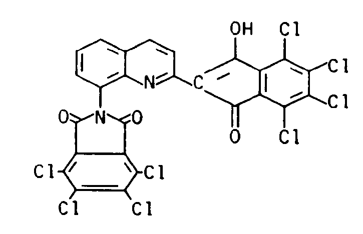

### Beispiel 7

Man verfuhr analog Beispiel 6, arbeitete jedoch wie folgt auf. Man kühlte das Reaktionsgemisch auf 120°C ab und versetzte es mit 500 g p-Xylol. Die resultierende Suspension wurde ausgetragen, das Pigment abfiltriert, mit 200 g p-Xylol gewaschen und getrocknet.

Man erhielt 79 g (95 %) Chinophthalon der Formel

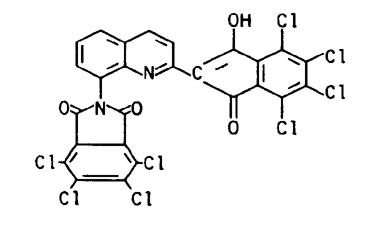

## Patentansprüche

1. Verfahren zur Herstellung von Chinophthalonen der Formel I 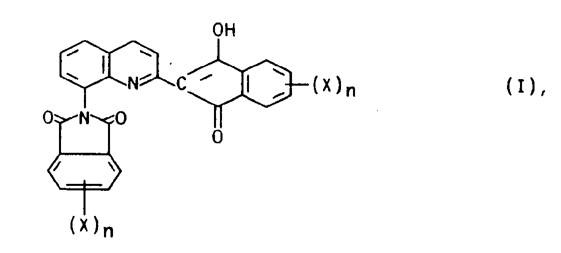 in der X Wasserstoff, Chlor oder Brom und n eine Zahl von 1 bis 4 bedeuten, durch Kondensation von 8-Aminochinaldin der Formel II 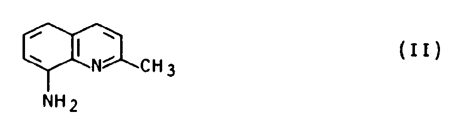 mit einem Phthalsäureanhydrid der Formel III 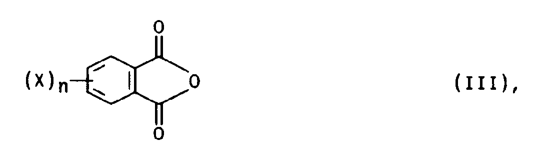 in der X und n jeweils die obengenannte Bedeutung besitzen, in Gegenwart eines Verdünnungsmittels, dadurch gekennzeichnet, daß man geschmolzene Benzoesäure als Verdünnungsmittel verwendet.

2. Verfahren nach Anspruch 1, dadurch gekennzeichnet, daß man die Kondensation bei einer Temperatur von 123 bis 200°C vornimmt.

3. Verfahren nach Anspruch 1, dadurch gekennzeichnet, daß man je Mol 8-Aminochinaldin 0,5 bis 20 Mol an Benzoesäure verwendet.

## Claims

1. A process for preparing quinophthalones of the formula I 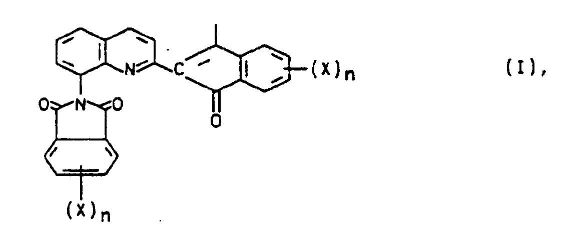 where X is hydrogen, chlorine or bromine and n is from 1 to 4, by condensation of 8-aminoquinaldine, which has the formula II 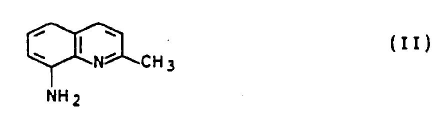 with a phthalic anhydride of the formula III 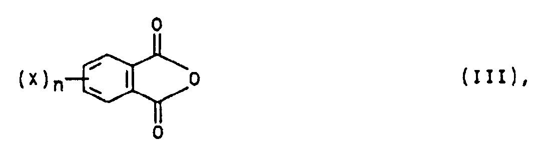 where x and n are each as defined above, in the presence of a diluent, characterized in that the diluent used is molten benzoic acid.

2. A process as claimed in claim 1, characterized in that the condensation is carried out at from 123 to 200°C.

3. A process as claimed in claim 1, characterized in that from 0.5 to 20 mol of benzoic acid is used per mole of 8-aminoquinaldine.

## Revendications

1. Procédé de préparation de quinophtalones de formule I 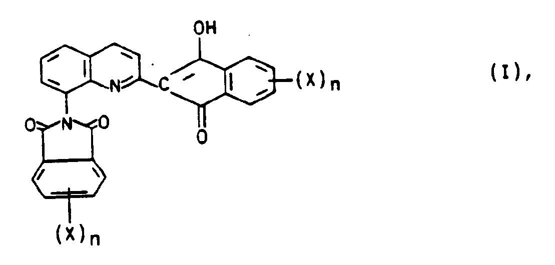 dans laquelle X représente un atome d'hydrogène, de chlore ou de brome et n un nombre de 1 à 4, par condensation d'une 8-aminoquinaldine de formule II 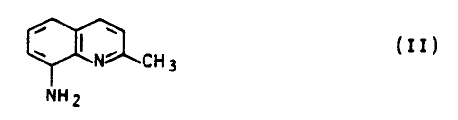 avec un anhydride phtalique de formule III 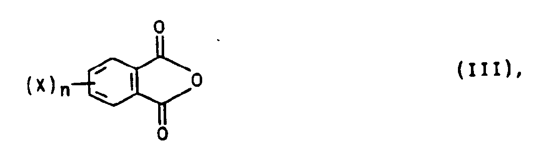 dans laquelle X et n ont chaque fois la signification sus-indiquée, en présence d'un diluant, caractérisé en ce que l'on utilise comme diluant de l'acide benzoïque fondu.

2. Procédé selon la revendication 1, caractérisé en ce que la condensation est effectuée à une température de 123 à 200°C.

3. Procédé selon la revendication 1, caractérisé en ce que l'on utilise 0,5 à 20 moles d'acide benzoïque par mole de 8-aminoquinaldine.
